(19) Europäisches Patentamt
European Patent Office
Office européen des brevets

(11) **EP 3 637 365 B1**

(12) **EUROPÄISCHE PATENTSCHRIFT**

(45) Veröffentlichungstag und Bekanntmachung des Hinweises auf die Patenterteilung:
**02.12.2020  Patentblatt 2020/49**

(51) Int Cl.:
***G06T 7/00*** *(2017.01)*    ***G06T 11/00*** *(2006.01)*

(21) Anmeldenummer: **18199866.7**

(22) Anmeldetag: **11.10.2018**

(54) **VERFAHREN ZUM BEWERTEN EINER VERLÄSSLICHKEIT EINES CT-VOLUMENBILDES, COMPUTERPROGRAMM UND COMPUTERLESBARES SPEICHERMEDIUM**

METHOD FOR RATING A RELIABILITY OF A CT VOLUME IMAGE, COMPUTER PROGRAM AND COMPUTER READABLE STORAGE MEDIUM

PROCÉDÉ D'ÉVALUATION D'UNE FIABILITÉ D'UNE IMAGE DE VOLUME CT, PROGRAMME INFORMATIQUE ET SUPPORT D'ENREGISTREMENT LISIBLE PAR ORDINATEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**15.04.2020  Patentblatt 2020/16**

(73) Patentinhaber: **Siemens Healthcare GmbH
91052 Erlangen (DE)**

(72) Erfinder:
- **Fischer, Peter
  91058 Erlangen (DE)**
- **Gemmel, Alexander
  91056 Erlangen (DE)**
- **Kleinszig, Gerhard
  91301 Forchheim (DE)**
- **Kreher, Björn
  91094 Bräuningshof (DE)**
- **Kunze, Holger
  91088 Bubenreuth (DE)**
- **Magaraggia, Jessica
  91058 Erlangen (DE)**
- **Schneider, Stefan
  91058 Erlangen (DE)**
- **Weiten, Markus
  90491 Nürnberg (DE)**

(56) Entgegenhaltungen:
- **SHEPP L A ET AL: "MAXIMUM LIKLIHOOD RECONSTRUCTION FOR EMISSION TOMOGRAPHY", IEEE TRANSACTIONS ON MEDICAL IMAGING, IEEE SERVICE CENTER, PISCATAWAY, NJ, US, Bd. MI-01, Nr. 2, Oktober 1982 (1982-10), Seiten 113-122, XP000877028, ISSN: 0278-0062**
- **LANGE K ET AL: "GLOBALLY CONVERGENT ALGORITHMS FOR MAXIMUM A POSTERIORI TRANSMISSION TOMOGRAPHY", IEEE TRANSACTIONS ON IMAGE PROCESSING, IEEE SERVICE CENTER, PISCATAWAY, NJ, US, Bd. 4, Nr. 10, Oktober 1995 (1995-10), Seiten 1430-1438, XP000537709, ISSN: 1057-7149, DOI: 10.1109/83.465107**
- **BAOYU DONG: "Expectation maximization reconstruction for circular orbit cone-beam CT", DESIGN FOR MANUFACTURABILITY THROUGH DESIGN-PROCESS INTEGRATION III, Bd. 6625, 9. September 2007 (2007-09-09), Seite 66251K, XP055570538, 1000 20th St. Bellingham WA 98225-6705 USA ISSN: 0277-786X, DOI: 10.1117/12.791222 ISBN: 978-1-5106-2781-9**

## Beschreibung

Die Erfindung betrifft ein Verfahren und ein System zum Bewerten einer Verlässlichkeit eines CT Volumenbildes, ein entsprechendes Computerprogrammprodukt und ein entsprechendes computerlesbares Speichermedium, auf dem ein solches Computerprogrammprodukt gespeichert ist.

Die Computertomographie (CT) ist heutzutage ein etabliertes bildgebendes Verfahren, mittels welchem ein Volumenbild, also ein 3D-Bild oder 3D-Bilddatensatz eines Untersuchungsobjekts, beispielsweise eines Patienten oder eines Gewebebereiches oder dergleichen, erzeugt werden kann. Dazu wird das Untersuchungsobjekt mittels Röntgenstrahlung durchstrahlt, welche von einer Strahlenquelle auf einer Seite des Untersuchungsobjekts ausgesendet und mittels eines Detektors auf einer gegenüberliegenden Seite des Untersuchungsobjekts detektiert wird. Weist ein dabei verwendetes Strahlenbündel eine zumindest im Wesentlichen kegelförmige oder pyramidenförmige Gestalt auf, so spricht man auch von Cone-Beam-CT. Es werden eine Vielzahl von 2D-Projektionsbildern des Untersuchungsobjekts aus verschiedenen Winkeln, also mit verschiedenen Winkelstellungen oder Angulationen des Strahlquelle-Detektor-Paares, also des Computertomographen, aufgenommen. Aus diesen Projektionsbildern wird dann das Volumenbild rekonstruiert.

Dabei können jedoch Bildartefakte auftreten, wodurch eine Bildqualität des Volumenbildes verschlechtert werden und dessen Aussagekraft für jeweilige relevante Fragestellungen leiden kann. Zur Verbesserung der Bildqualität können beispielsweise Methoden oder Verfahren eingesetzt werden, die zumindest zum Teil auf Basis von Vorwissen Modifikationen oder Anpassungen automatisch durchführen. Zum Beispiel kann dieses Vorwissen bei Verfahren, die auf der bayesschen Statistik beruhen, durch die a-priori Wahrscheinlichkeitsverteilung beschrieben und bei der Maximierung der Gesamtwahrscheinlichkeit berücksichtigt werden. Ebenso sind heutzutage auf deep learning (deutsch etwa "tiefgehendes Lernen") basierende Ansätze bekannt. Dabei wird das Vorwissen für ein jeweils aktuelles Problem aus einer großen Menge von annotierten Trainingsdaten extrahiert, beispielsweise im Rahmen eines Lern- oder Trainingsprozesses eines neuronalen Netzes. Derartige Ansätze haben in vielen Bereichen eine sehr gute Fähigkeit gezeigt, gelernte Informationen oder gelerntes Vorwissen auf ein jeweils aktuelles Problem oder Ergebnis zu übertragen und damit beispielsweise die Bildqualität von CT-Volumenbildern zu verbessern. Es können beispielsweise ein Rauschverhalten verbessert, eine Generalisierung bei unvollständig rekonstruierten Schichtbildern (Tomosynthese) erreicht oder Streu- und Bewegungsartefakte reduziert werden.

Es kann jedoch problematisch sein, dass ein Nutzer oder Betrachter eines entsprechend rekonstruierten und gegebenenfalls modifizierten CT-Volumenbildes nicht erkennen kann, welcher Anteil des jeweiligen Volumenbildes auf tatsächlich gemessenen, patientenspezifischen Daten beruht und welcher Anteil aus dem Vorwissen abgeleitet oder modifiziert wurde, also nicht direkt aus den gemessenen Projektionsbildern resultiert oder in diesen enthalten ist. Dieser letztere, aus dem Vorwissen abgeleitete Anteil kann zwar im Sinne des Vorwissens die wahrscheinlichste Realisierung darstellen. In einem konkreten Einzelfall kann aber nicht notwendigerweise davon ausgegangen werden, dass dieser Anteil beziehungsweise das zugrunde liegende Vorwissen mit der Realität des Einzelfalls, also mit der tatsächlichen Anatomie des Patienten übereinstimmt. Es kann dementsprechend bei der Verwendung von derartigen Bildverbesserungsverfahren zur Verbesserung der Bildqualität passieren, dass ein CT-Volumenbild erzeugt wird, welches zumindest in Details nicht mit der realen physischen Anatomie des Patienten übereinstimmt.

Der Artikel von Lange und Fessler: "Globally Convergent Algorithms for Maximum a Posteriori Transmission Tomography",IEEE TRANSACTIONS ON IMAGE PROCESSING, IEEE SERVICE CENTER, PISCATAWAY, NJ, US, Bd. 4, Nr. 10, Oktober 1995, S. 1430-1438, offenbart ein iteratives Rekonstruktionsverfahren für Transmissions-CT, in welchem eine Likelihood-Funktion des aktuellen Bildes mit Hinblick auf die gemessenen Projektionsdaten optimiert wird. Der Algorithmus liefert eine monotone Steigerung der Likelihood-Funktion mit jeder Iteration.

Aufgabe der vorliegenden Erfindung ist es, eine Praxistauglichkeit von Verfahren zur automatischen Verbesserung einer Bildqualität zu erhöhen. Diese Aufgabe wird erfindungsgemäß durch die Gegenstände der unabhängigen Patentansprüche gelöst. Vorteilhafte Ausgestaltungen und Weiterbildungen der vorliegenden Erfindung sind in den abhängigen Patentansprüchen, in der Beschreibung und in den Figuren angegeben.

Vorliegend ist dazu ein erfindungsgemäßes Verfahren zum Bewerten einer Verlässlichkeit von CT-Volumenbildern oder einzelnen Merkmalen eines oder mehrere CT-Volumenbilder eines Untersuchungsobjekts vorgesehen. Das Untersuchungsobjekt kann ein Patient oder ein Organ oder Teilbereich des Patienten ebenso sein wie ein anderer, der Computertomographie zugänglicher Gegenstand. In einem Verfahrensschritt des Verfahrens wird ein aus gemessenen Projektionsbildern des Untersuchungsobjekts rechnerisch rekonstruiertes erstes CT-Volumenbild erfasst. Die Bezeichnung "erstes CT-Volumenbild" dient hier lediglich als Name und bezieht sich nicht notwendigerweise auf einen Rekonstruktions- oder Erfassungszeitpunkt dieses CT-Volumenbildes. Die gemessenen Projektionsbilder sind dabei 2D-Röntgenbilder des Untersuchungsobjekts, die aus verschiedenen Winkeln oder Projektionsrichtungen aufgenommen wurden. Das Erfassen des CT-Volumenbildes kann hier bedeuten, dass das bereits fertig rekonstruierte erste CT-Volumenbild durch eine für ein Ausführen des erfindungsgemäßen Verfahrens eingerichtetes System oder eine entsprechende Datenverarbeitungseinrichtung von einem damit verbundenen Datenspeicher oder Speichermedium, beispielsweise einer Computertomographieanlage, abgerufen wird. Ebenso kann das Erfassen beispielsweise ein Messen oder Aufnehmen der Projektionsbilder und/oder das Rekonstruieren des ersten CT-Volumenbildes bedeuten oder umfassen. Es kann insbesondere auch wenigstens eines der gemessenen Projektionsbilder erfasst, also dem System oder der Datenverarbeitungseinrichtung für das Verfahren zum Verarbeiten oder Verwenden bereitgestellt werden.

In einem weiteren Verfahrensschritt des erfindungsgemäßen Verfahrens wird ein zur Bildartefaktreduktion mittels eines Bildverbesserungsverfahrens erzeugtes modifiziertes CT-Volumenbild desselben Untersuchungsobjekts erfasst. Das Bildverbesserungsverfahren kann beispielsweise eines der eingangs genannten Verfahren sein oder umfassen und dient zum Verbessern einer Bildqualität des ersten CT-Volumenbildes beziehungsweise des modifizierten CT-Volumenbildes. Das erste CT-Volumenbild und das modifizierte CT-Volumenbild stellen also dasselbe oder ein übereinstimmendes Volumen, das heißt denselben oder einen übereinstimmenden Bereich des Untersuchungsobjekts dar, bilden diesen also ab. Das modifizierte CT-Volumenbild kann durch Modifizieren des rekonstruierten ersten CT-Volumenbildes erzeugt werden. Dabei ist zu beachten, dass bereits beim Rekonstruieren des ersten CT-Volumenbildes ein, wie einleitend beschrieben, auf Vorwissen basierendes Bildverbesserungsverfahren eingesetzt werden kann. Dies kann grundsätzlich dasselbe Bildverbesserungsverfahren sein, welches auch für das modifizierte CT-Volumenbild verwendet wird. Es können jedoch beispielsweise andere Parameterwerte verwendet werden. Ebenso kann das modifizierte CT-Volumenbildes unter Verwendung des Bildverbesserungsverfahrens direkt aus den gemessenen Projektionsbildern erzeugt oder rekonstruiert werden, insbesondere also unter Berücksichtigung von Vorwissen über das Untersuchungsobjekt, über eine jeweilige Aufnahmesituation und/oder über die jeweilige Rekonstruktionsaufgabe. In jedem Fall unterscheidet sich das modifizierte CT-Volumenbild von dem ersten CT-Volumenbild. Insbesondere kann für das modifizierte CT-Volumenbild wenigstens eine Variation oder Alternative einer Bildverbesserung oder Anpassung verwendet werden, welche nicht für das erste CT-Volumenbild verwendet wurde. Eine Modifikation oder Anpassung kann beispielsweise ein Fortsetzen eines Verlaufs eines anatomischen Merkmals über einen gestörten oder nicht aufgenommenen Bildbereich hinweg und/oder ein Verändern eines Intensitäts- oder Grauwerte, beispielsweise an einem Störpeak, sein oder umfassen. Das Erfassen des modifizierten CT-Volumenbildes kann wie für das erste CT-Volumenbild erläutert ein Abrufen des modifizierten CT-Volumenbildes aus einem Datenspeicher bedeuten oder umfassen. Ebenso kann das Erzeugen oder Rekonstruieren des modifizierten CT-Volumenbildes Teil des erfindungsgemäßen Verfahrens sein.

In einem weiteren Verfahrensschritt des erfindungsgemäßen Verfahrens wird wenigstens eine Projektionsrichtung bestimmt, für die ein in dieser Projektionsrichtung verlaufendes gedachtes oder simuliertes Strahlenbündel wenigstens ein im Vergleich zu dem ersten CT-Volumenbild modifiziertes Voxel des modifizierten CT-Volumenbildes durchdringt. Als diese Projektionsrichtung kann dabei bevorzugt eine Projektionsrichtung bestimmt werden, welche für das Messen oder Aufnehmen der Projektionsbilder verwendet wurde, für die also ein gemessenes Projektionsbild des Untersuchungsobjekts existiert, insbesondere erfasst wurde.

In einem weiteren Verfahrensschritt des erfindungsgemäßen Verfahrens wird für die wenigstens eine bestimmte Projektionsrichtung aus dem ersten CT-Volumenbild und aus dem modifizierten CT-Volumenbild jeweils wenigstens ein digital rekonstruiertes Röntgenbild (DRR) berechnet. Das oder die aus dem ersten CT-Volumenbild und aus dem modifizierten CT-Volumenbild berechneten beziehungsweise simulierten digital rekonstruierten Röntgenbilder zeigen also denselben oder einen übereinstimmenden Bereich oder Ausschnitt des Untersuchungsobjektes aus derselben Blickrichtung, haben also den gleichen oder einen übereinstimmenden Bildausschnitt. Aufgrund der Art, wie die dafür verwendete Projektionsrichtung bestimmt, also ausgewählt wurde, fließt in das aus dem modifizierten CT-Volumenbild berechneten digital rekonstruierten Röntgenbild zumindest der eine modifizierte Voxel, also eine durch das Bildverbesserungsverfahren vorgenommene Maßnahme zur Verbesserung der Bildqualität, ein. Ebenso fließt der entsprechende Voxel ohne diese Modifikation in das aus dem ersten CT-Volumenbild berechnete oder simulierte digital rekonstruierten Röntgenbild ein. Dadurch sind die digital rekonstruierten Röntgenbilder also sinnvoll miteinander vergleichbar.

In einem weiteren Verfahrensschritt wird eine Ähnlichkeit bestimmt zwischen dem wenigstens einen aus dem ersten CT-Volumenbild berechneten digital rekonstruierten Röntgenbild und dem jeweils entsprechenden gemessenen Projektionsbild oder den entsprechenden Teilen mehrerer der gemessenen Projektionsbilder. Letzteres kann der Fall sein, wenn die bestimmte Projektionsrichtung für die DRRs nicht einer Projektionsrichtung entspricht, welche für eines der gemessenen Projektionsbilder verwendet wurde. Weiter wird in diesem Verfahrensschritt eine Ähnlichkeit zwischen dem wenigstens einen aus dem modifizierten CT-Volumenbild berechneten digital rekonstruierten Röntgenbild und dem jeweils entsprechenden gemessenen Projektionsbild oder den entsprechenden Teilen mehrerer der gemessenen Projektionsbilder bestimmt. Mit anderen Worten wird also bestimmt oder berechnet, inwieweit oder wie stark, also in welchem Umfang oder Ausmaß sich die digital rekonstruierten Röntgenbilder von dem oder den entsprechenden gemessenen Projektionsbildern unterscheiden. Idealerweise würde ein digital rekonstruiertes Röntgenbild dem korrespondierenden, also in der gleichen Projektionsrichtung aufgenommenen gemessenen Projektionsbild entsprechen. Dies ist in der Praxis aber in der Regel nicht zu erwarten, beispielsweise aufgrund von Eigenschaften eines zum Rekonstruieren der CT-Volumenbilder verwendeten Verfahrens oder Modells, des Bildverbesserungsverfahrens, dabei gemachter Annahmen und/oder einer begrenzten Rechengenauigkeit oder dergleichen. Da sich zudem das erste CT-Volumenbild und das modifizierte CT-Volumenbild per Definition voneinander unterscheiden, ist auch zu erwarten, dass die aus diesen CT-Volumenbildern berechneten digital rekonstruierten Röntgenbilder unterschiedliche Ähnlichkeiten zu dem entsprechenden gemessenen Projektionsbild aufweisen, diesem also unterschiedlich ähnlich oder nahe sind.

In einem weiteren Verfahrensschritt des erfindungsgemäßen Verfahrens werden zum Bewerten der Verlässlichkeit der CT-Volumenbilder die bestimmten Ähnlichkeiten miteinander verglichen. Zumindest implizit kann damit ebenso die Verlässlichkeit der durch das Bildverbesserungsverfahren für das modifizierte CT-Volumenbild vorgenommenen Modifikationen oder Änderungen, des für das Rekonstruieren der CT-Volumenbilder verwendeten Verfahrens oder Modells oder einer dabei verwendeten Annahme bestimmt oder bewertet werden. Es kann hier die Verlässlichkeit des rekonstruierten ersten CT-Volumenbildes und/oder des modifizierten CT-Volumenbildes bestimmt und/oder bewertet werden. Ist beispielsweise die Ähnlichkeit zwischen dem aus dem ersten CT-Volumenbild berechneten DRR und dem entsprechenden gemessenen Projektionsbild signifikant größer als die Ähnlichkeit zwischen dem gemessenen Projektionsbild und dem aus dem modifizierten CT-Volumenbild berechneten DRR, so kann dies als Indiz dafür dienen, dass das erste CT-Volumenbild beziehungsweise dass zum Rekonstruieren des ersten CT-Volumenbildes aus den gemessenen Projektionsbildern verwendete Verfahren oder Modell relativ verlässlich ist. Im Umkehrschluss kann dies ebenso bedeuten, dass das Vorwissen, welches für das Erzeugen des modifizierten CT-Volumenbildes explizit oder implizit verwendet oder zugrunde gelegt wurde, zumindest für den jeweiligen Einzelfall keine verlässliche Beschreibung des jeweiligen realen Untersuchungsobjekts darstellt oder ermöglicht. Es kann dann also ein Widerspruch zwischen dem Vorwissen und der Messung, also den - typischerweise als artefaktfrei angenommenen - gemessenen Projektionsbildern, bestehen.

Die Verlässlichkeit eines CT-Volumenbildes, also eine Konfidenz für oder in dieses CT-Volumenbild gibt im Sinne der vorliegenden Erfindung insbesondere an, wie wahrscheinlich es ist, dass das jeweilige rechnerisch rekonstruierte CT-Volumenbild der Realität, also der realen physischen Anatomie des Untersuchungsobjekts entspricht. Die Verlässlichkeit oder Konfidenz kann für den wenigstens einen modifizierten Voxel oder global für das jeweilige gesamte CT-Volumenbild, also das gesamte rekonstruierte oder modifizierte Volumen beziehungsweise für die verwendete Rekonstruktionsmethode oder das verwendete Bildverbesserungsverfahren angegeben werden. Die Verlässlichkeit kann dabei unterschiedlich ausgedrückt werden, beispielsweise als Zahlenwert oder als Prozentsatz oder durch eine qualitative Angabe dazu, ob das erste CT-Volumenbild oder das modifizierte CT-Volumenbild verlässlicher ist, also mit größerer Wahrscheinlichkeit der Realität entspricht. Diese Angaben können beispielsweise direkt aus einem Vergleichsergebnis der bestimmten Ähnlichkeiten abgeleitet werden.

Das erfindungsgemäße Verfahren ist vorteilhaft unabhängig von der tatsächlich verwendeten Rekonstruktions- oder Bildverbesserungsmethode und kann einem Nutzer oder Anwender, beispielsweise einem Arzt, ermöglichen, zu entscheiden, ob er eine bestimmte medizinische Fragestellung oder Diagnose auf Basis der ihm vorliegenden CT-Volumenbilder beantworten beziehungsweise stellen kann oder ob beispielsweise weitere Untersuchungen geboten sind. Ebenso kann der Anwender durch das erfindungsgemäße Verfahren vorteilhaft dabei unterstützt werden, zu entscheiden, ob eine bestimmte Bildverbesserungsmethode oder ein bestimmtes Rekonstruktionsverfahren im Einzelfall angewendet werden soll oder nicht. Da die Bewertung der CT-Volumenbilder mittels der vorliegenden Erfindung unabhängig von deren Generierung, also unabhängig von der Bildverbesserung und der Rekonstruktion, ist, kann die vorliegende Erfindung vorteilhaft als Wegbereiter (englisch: enabler) beispielsweise für viele aktuell entwickelte oder in der Entwicklung befindliche deep learning-basierte Verfahren insbesondere im klinischen oder medizinischen Umfeld dienen. Da durch die vorliegende Erfindung eine Bewertung der Verlässlichkeit vorliegt und eine einfache Validierung möglich ist, da das beispielsweise ein auf konventionelle Weise, also ohne Verwendung einer deep learning-basierten Methode, rekonstruiertes CT-Volumenbild - welches das erste CT-Volumenbild oder hiervon verschieden sein kann - als Referenz oder Rückfallmöglichkeit verwendet werden kann, kann so eine Verbreitung, Akzeptanz und praktische Anwendung beziehungsweise Anwendbarkeit von auf Vorwissen basierenden Rekonstruktions- und/oder Bildverbesserungsverfahren vorteilhaft gefördert werden.

In vorteilhafter Ausgestaltung der vorliegenden Erfindung wird bereits das erste CT-Volumenbild als durch auf Vorwissen basierende Abschätzungen für eine verbesserte Bildqualität angepasstes CT-Volumenbild rekonstruiert. Das modifizierte CT-Volumenbild wird dann durch lokale Modifikationen aus dem ersten CT-Volumenbild erzeugt. Mit anderen Worten enthält dann also bereits das erste CT-Volumenbild eine Abschätzung, welche nicht notwendigerweise direkt in den gemessenen Projektionsbildern enthalten oder erkennbar ist. Eine solche Abschätzung kann insbesondere eine wahrscheinlichste Variante sein, wie ein bestimmtes Merkmal gemäß dem Vorwissen auch bei dem jeweiligen aktuellen Untersuchungsobjekt real aussieht. Beispielsweise kann ein als solches identifiziertes Bildartefakt oder ein Bildfehler durch eine solche Abschätzung ersetzt oder eine Aufnahmelücke durch eine solche Abschätzung aufgefüllt werden. Dass das modifizierte CT-Volumenbild lokale Modifikationen gegenüber dem ersten CT-Volumenbild aufweist, bedeutet hier insbesondere, dass grundsätzlich das gleiche Rekonstruktions- oder Bildverbesserungsverfahren für das modifizierte CT-Volumenbild verwendet wird wie für das erste CT-Volumenbild. Das modifizierte CT-Volumenbild und das erste CT-Volumenbild können also zu einem signifikanten Anteil identisch sein. Die lokalen Modifikationen betreffen nur einen oder mehrere Teilbereiche der CT-Volumenbilder, welche beispielsweise nur wenige Prozent des Bildvolumens ausmachen können und von nicht-modifizierten Volumendaten oder Teilbereichen umgeben sein können.

In einer alternativen vorteilhaften Ausgestaltung der vorliegenden Erfindung werden zum Rekonstruieren des ersten CT-Volumenbildes nur direkt in den gemessenen Projektionsdaten enthaltene Informationen und keine darüber hinausgehenden Abschätzungen verwendet werden, derartige Abschätzungen aber zum Erzeugen des modifizierten CT-Volumenbildes durch das Bildverbesserungsverfahren angewendet. Hier wird das erste CT-Volumenbild also möglichst genau den tatsächlich gemessenen Daten, also den gemessenen Projektionsbildern, entsprechend oder folgend rekonstruiert. Dabei kann einen suboptimale Bildqualität in Kauf genommen werden, um vorteilhaft das CT-Volumenbild als Referenz- oder Vergleichswert zum Bewerten der Abschätzungen oder Bildverbesserungen oder des Vorwissens verwenden zu können.

In vorteilhafter Ausgestaltung der vorliegenden Erfindung werden die Ähnlichkeiten mittels einer Likelihood-Funktion charakterisiert. Zum Bewerten der Verlässlichkeit wird mittels der Likelihood-Funktion jeweils eine Likelihood für das erste CT-Volumen und für das modifizierte CT-Volumen berechnet. Die Likelihood ist also ein entsprechender Funktionswert der Likelihood-Funktion. Die Likelihood wird dabei für ein CT-Volumenbild maximal wenn ein daraus berechnetes digital rekonstruiertes Röntgenbild und das entsprechende gemessene Projektionsbild zueinander identisch sind. Die Likelihood-Funktion drückt mit anderen Worten also die Ähnlichkeit zwischen Simulation und akquirierten Daten, das heißt zwischen den DRRs und dem entsprechenden gemessenen Projektionsbild aus und bewertet einen entsprechenden Fehler zwischen den simulierten oder berechneten DRRs und dem entsprechenden gemessenen Projektionsbild. Es kann vorteilhaft dieselbe Methode oder dasselbe Verfahren zum Berechnen aller der digital rekonstruierten Röntgenbilder verwendet werden. Der Fehler kann dann insbesondere zurückgeführt werden auf einen bei dem Rekonstruieren der CT-Volumenbilder und/oder auf die durch die Bildverbesserungsmethode vorgenommene Modifikation. Es kann hier eine nicht-normalisierte Likelihood-Funktion verwendet werden. Sofern verfügbar, kann aber ebenso eine normalisierte Likelihood-Funktion verwendet werden.

Der Mechanismus oder Formalismus einer Likelihood-Funktion ist als mathematische Methode hinreichend bekannt. Deren Anwendung in der vorliegend erfindungsgemäßen Art und Weise mit einer Ausprägung oder Realisierung der CT-Volumenbilder als variierter Parameter $\vartheta$ eröffnet vorteilhaft jedoch gänzlich neue Möglichkeiten für die praktische medizinische Bildgebung und darauf basierende Bewertungen oder Diagnosen. Als der Parameter $\vartheta$ für die Likelihood-Funktion wird hier also eine Variation eines Merkmals oder Features oder dessen Realisierung in dem oder den rekonstruierten CT-Volumenbildern verwendet, welches beziehungsweise welche mittels der Bildverbesserungsmethode modifiziert oder variiert wird.

Unterschiedliche Modifikationen, also Realisierungen oder Darstellungen des jeweiligen Merkmals in den rekonstruierten CT-Volumenbilder können dabei unterschiedlich wahrscheinlich sein, also mit unterschiedlicher Wahrscheinlichkeit der physischen Realität des Patienten entsprechen und im Formalismus der Likelihood-Funktion somit unterschiedliche Likelihoods aufweisen. Die Verwendung der Likelihood-Funktion, beispielsweise als Grundlage für die Maximum-Likelihood-Methode, bietet vorteilhaft eine Möglichkeit, die Verlässlichkeit auf objektive und mathematisch fundierte Weise zu bestimmen beziehungsweise zu bewerten. Damit wird vorteilhaft auch eine Vergleichsgrundlage für verschiedene Rekonstruktions- und Bildverbesserungsverfahren geschaffen.

In vorteilhafter Weiterbildung der vorliegenden Erfindung wird ein Rauschen oder ein Rauschwert $\sigma$ des wenigstens einen gemessenen Projektionsbildes bestimmt. Die Verlässlichkeit wird dann in Abhängigkeit davon bewertet, ob eine Differenz zwischen der für das erste CT-Volumenbild berechneten Likelihood $L(\vartheta_{orig})$ und der für das modifizierte CT-Volumenbild berechneten Likelihood $L(\vartheta_{mod})$ größer oder kleiner ist als das Rauschen $\sigma$. Es bieten sich hier also durch das Heranziehen oder Verwenden des Rauschens je nach Fall oder Situation unterschiedliche Interpretationsmöglichkeiten für einen Effekt, den die durch das Bildverbesserungsverfahren bewirkte Modifikationen in dem modifizierten CT-Volumenbild beziehungsweise dessen lokale Modifikationen oder Anpassungen gegenüber dem gegebenenfalls bereits mittels des oder eines Bildverbesserungsverfahrens erzeugten ersten CT-Volumenbild haben auf die Likelihood $L(\vartheta_{orig})$ des ersten CT-Volumenbildes oder relativ zu diesem oder auf eine Likelihood $L(\vartheta_{mod})$ des modifizierten CT-Volumenbildes. Es werden hier der Verständlichkeit halber lediglich die vollständigen CT-Volumenbilder genannt, wobei die Likelihoods sich jedoch auf einzelne Merkmale, Bildbereiche, Bildverbesserungen oder Modifikationen beziehen können.

Gilt beispielsweise

$$L(\vartheta_{orig}) - L(\vartheta_{mod}) > \sigma \quad ,$$

so führt die Modifikation zu einer Erniedrigung des Likelihood, das modifizierte CT-Volumenbild entspricht also mit geringerer Wahrscheinlichkeit der Realität. Dementsprechend wird hierdurch ein jeweiliges Merkmal des ersten CT-Volumenbildes bestätigt, da es eine größere Likelihood und somit eine größere Verlässlichkeit als die modifizierte Version, Variante oder Realisierung dieses Merkmals in dem modifizierten CT-Volumenbild aufweist. Mit anderen Worten führt in diesem Fall beispielsweise eine lokale Modifikation oder eine lokale Änderung einer Realisierung einer Bildverbesserung des ersten CT-Volumenbildes, die ausgehend von dem ersten CT-Volumenbild vorgenommen wurde, um das modifizierte CT-Volumenbild zu erhalten, zu einer signifikanten Verringerung der Likelihood. Es kann daraus geschlossen werden, dass die Modifikation oder Änderung, also ein geänderter Aspekt, von den gemessenen Daten unterstützt wird. Damit besteht eine relativ hohe Konfidenz in die Modifikation oder Änderung.

Gilt hingegen

$$L(\vartheta_{mod}) - L(\vartheta_{orig}) > \sigma \quad ,$$

so führt die Modifikation durch das Bildverbesserungsverfahren zu einer Erhöhung der Likelihood. Das modifizierte CT-Volumenbild kann dann mit größerer Wahrscheinlichkeit der Realität entsprechen als das erste CT-Volumenbild. Dementsprechend kann dann das nicht modifizierte Merkmal in dem ersten CT-Volumenbild als widersprüchlich zu der Messung, also zu den gemessenen Projektionsbildern, aufgefasst oder interpretiert werden. Die Wahrscheinlichkeit dafür, dass die Realität von dem Merkmal in der in dem ersten CT-Volumenbild realisierten oder dargestellten Weise oder Variante abweicht, ist also relativ hoch. Somit ist im Umkehrschluss die Verlässlichkeit des ersten CT-Volumenbildes dann also relativ niedrig, insbesondere niedriger als die Verlässlichkeit des modifizierten CT-Volumenbildes. Mit anderen Worten führt in diesem Fall die lokale Modifikation oder Änderung zu einer signifikanten Erhöhung der Likelihood. Es kann daraus geschlossen werden, dass der entsprechende geänderte oder angepasste Aspekt von den gemessenen Daten nicht unterstützt wird oder sogar widersprüchlich zu diesen ist. Damit ist dann eine relativ geringe Konfidenz für die Modifikation oder Änderung gegeben.

In einem dritten Fall kann

$$| L(\vartheta_{orig}) - L(\vartheta_{mod}) | \leq \sigma$$

gelten. In diesem Fall führt die Modifikation durch das Bildverbesserungsverfahren zu keiner signifikanten Veränderung des Likelihood. Ein jeweiliges Merkmal in dem ersten CT-Volumenbild oder in dem modifizierten CT-Volumenbild wird dann also von den akquirierten Daten, also von den gemessenen Projektionsbildern nicht explizit bestätigt. Dies kann darauf zurückzuführen sein, dass das entsprechende Merkmal nicht gemessen wurde, beispielsweise bei Verwendung eines Tomosynthese-Verfahrens, oder es kann bedeuten, dass das Rauschen $\sigma$ in dem gemessenen Projektionsbild zu groß ist. Es kann mit anderem Worten in diesem Fall geschlossen werden, dass die Modifikation oder Änderung nicht in den gemessenen Daten abgebildet ist. Damit ist für die Modifikation oder Änderung ebenfalls eine relativ geringe Konfidenz, beispielsweise eine Konfidenz nahe null, dafür gegeben, dass der geänderte oder angepasste Aspekt in den gemessenen Daten enthalten ist, also durch diese gestützt wird.

Eine Veränderung der Likelihood wird im Sinne der vorliegenden Veränderung also erst dann als signifikant eingestuft, wenn sie betragsmäßig größer als das Rauschen $\sigma$ des jeweiligen gemessenen Projektionsbildes oder größer als eine aufgrund des Rauschens $\sigma$ ohnehin zu erwartende Variation oder Unsicherheit der Likelihood oder der Ähnlichkeit ist.

Da die beschriebenen Differenzen, also Änderungen der Likelihood einem Betrag eines lokalen Gradienten der Likelihood-Funktion entsprechen können, können hier vorteilhaft Methoden verwendet werden, die beispielsweise bereits bei einer nichtrigiden Registrierung Einsatz finden. Auch ein Markov-Ketten-Monte-Carlo-Verfahren kann vorteilhaft eingesetzt werden, um relativ komplexe Verteilungen erfassen und analysieren zu können. Mit anderen Worten können diese oder derartige Verfahren oder Methoden verwendet werden, um beispielsweise eine lokale Modifikation oder Anpassung auf das - gegebenenfalls bereits mittels des Bildverbesserungsverfahrens basierend auf Vorwissen verbesserte - erste CT-Volumenbild anzuwenden, um den Gradienten der Likelihood oder Likelihood-Funktion zu bestimmen.

Die hier beschriebene Verwendung des Rauschens oder Rauschwertes $\sigma$ als Referenz oder Vergleichswert bietet vorteilhaft die Möglichkeit, eine Bewertung oder Interpretation auf besonders einfache Art und Weise und inhärent an jeweilige im Einzelfall gegebene Bedingungen angepasst vorzunehmen.

In weiterer vorteilhafter Ausgestaltung der vorliegenden Erfindung wird für das modifizierte CT-Volumen oder CT-Volumenbild und/oder für das rekonstruierte erste CT-Volumen oder CT-Volumenbild eine Konfidenzkarte erzeugt, welche zumindest für jedes durch das Bildverbesserungsverfahren modifizierte Voxel dessen Verlässlichkeit angibt. In der Konfidenzkarte sind also Konfidenz- oder Verlässlichkeitswerte für den oder die modifizierten Voxel beziehungsweise diesen Voxeln entsprechende Voxel des ersten CT-Volumenbildes, also für ein jeweils dargestelltes oder rekonstruiertes Merkmal dargestellt oder eingetragen. Die Konfidenzkarte ist hier also Voxel-genau, während die Verlässlichkeit insgesamt aber ebenso als globaler Gesamtwert für das rekonstruierte Volumen, also für das erste CT-Volumenbild und/oder für das modifizierte CT-Volumenbild insgesamt angegeben sein kann. Das hier beschriebene Verfahren ermöglicht vorteilhaft also unabhängig von der eigentlichen Bildverbesserungsmethode, die Konfidenzkarte für das jeweils betrachtete Volumen zu erstellen. Die Konfidenzkarte kann dann dem jeweiligen Anwender, beispielsweise einem Arzt oder Mediziner, transparent gemacht, also ausgegeben oder dargestellt werden. Mit diesen zusätzlichen Informationen oder Daten kann der Arzt dann vorteilhaft besonders zuverlässig entscheiden, ob er eine bestimmte Fragestellung auf Basis des ersten CT-Volumenbildes oder auf Basis des modifizierten CT-Volumenbildes beantworten kann oder nicht.

Durch die Konfidenzkarte, die beispielsweise dem jeweiligen CT-Volumenbild, insbesondere ein- und ausschaltbar, überlagert werden kann, kann der Anwender vorteilhaft besonders genau und einfach erkennen, in welchen Bildbereichen des jeweiligen CT-Volumenbildes eine für seine jeweiligen Anforderungen ausreichende beziehungsweise zu große oder zu kleine Konfidenz in die Darstellung oder Rekonstruktion gegeben ist. Die Pixel- beziehungsweise Voxel-genaue Konfidenzkarte ist dabei besonders vorteilhaft, da durch das Rekonstruktionsverfahren oder die verwendete Bildverbesserungsverfahren in unterschiedlichen Bildbereichen unterschiedliche und/oder unterschiedlich starke Artefakte oder Modifikationen eingebracht oder erzeugt werden können. Dies kann beispielsweise abhängig sein von lokalen Intensitätsvariationen, also einer jeweiligen lokalen Gewebeart oder Gewebezusammensetzung oder dergleichen mehr. Es kann also sein, dass in einem lokal begrenzten Teilbereich eine Modifikationen vorgenommmen wurde, die dort zu einer verringerten Likelihood führt, in einem anderen lokalen Bildbereich jedoch keine Modifikation oder eine Modifikation, die dort zu einer Erhöhung der Likelihood führt, vorgenommen wurde. Je nachdem welcher Bildbereich im jeweiligen Einzelfall beispielsweise für eine Diagnose relevant ist, kann dann mittels der Konfidenzkarte besonders einfach und zuverlässig eine optimale Auswahl des zu verwendenden CT-Volumenbildes getroffen werden.

In vorteilhafter Weiterbildung der vorliegenden Erfindung werden die in der Konfidenzkarte eingetragenen Verlässlichkeiten oder Konfidenzwerte nach einem vorgegebenen Schema gemäß ihren Größen oder Werten farbcodiert. Dabei kann eine vorgegebene absolute oder relative Skala verwendet oder zugrunde gelegt werden. Mit anderen Worten wird die Konfidenzkarte also als Heatmap erzeugt. Dies kann vorteilhaft eine besonders leicht, schnell und intuitiv erfassbare Interpretation der Konfidenzkarte auch für ungeübtes oder ungeschultes Personal ermöglichen.

In weiterer vorteilhafter Ausgestaltung der vorliegenden Erfindung wird automatisch ein Hinweis oder eine Warnung an einen Benutzer ausgegeben, falls die Verlässlichkeit kleiner als ein vorgegebener Schwellenwert ist. Dadurch kann vorteilhaft ein erhöhter Benutzungskomfort erreicht werden, da im Falle einer oberhalb des vorgegebenen Schwellenwertes liegenden Verlässlichkeit beispielsweise keine weiteren Maßnahmen oder Eingaben von Seiten des Benutzers erforderlich sind oder abgefragt werden müssen. Für den Fall dass die Verlässlichkeit kleiner ist als der vorgegebene Schwellenwert, kann durch den automatisch ausgegebenen Hinweis oder die automatisch ausgegebene Warnung hingegen vorteilhaft besonders zuverlässig die Wahrscheinlichkeit dafür verringert werden, dass der jeweilige Benutzer oder Anwender auf Basis eines entsprechend wenig verlässlichen CT-Volumenbildes eine gegebenenfalls aus medizinischer Sicht kritische Entscheidung trifft, insbesondere ohne dass ihm die entsprechend geringe Verlässlichkeit bekannt wäre. Der Hinweis beziehungsweise die Warnung kann beispielsweise automatisch durch ein zum Durchführen des erfindungsgemäßen Verfahrens eingerichtetes beziehungsweise verwendetes System erzeugt und ausgegeben werden. Das System kann dazu beispielsweise eine Datenverarbeitungseinrichtung zum Verarbeiten der gemessenen Projektionsbildern und/oder des ersten CT-Volumenbildes umfasse, welche ihrerseits einen Datenspeicher aufweisen kann, in welchem verschiedene Rekonstruktions- oder Bildverbesserungsverfahren, also entsprechende Programme oder Programmmodule hinterlegt sind. Durch die automatische Auswahl und/oder Verwendung der alternativen Methode oder Methoden, als des alternativen Rekonstruktions- oder Bildverbesserungsverfahrens, kann vorteilhaft ein verbesserter Bedien- oder Nutzungskomfort für einen jeweiligen Anwender erreicht werden.

Ein weiterer Aspekt der vorliegenden Erfindung ist ein Computerprogramm oder Computerprogrammprodukt, welches Befehle oder Instruktionen umfasst, die bei einer Ausführung des Computerprogramms durch einen Computer diesen dazu veranlassen, zumindest eine Ausführungsform des erfindungsgemäßen Verfahrens, insbesondere automatisch oder semiautomatisch, auszuführen. Der Computer kann insbesondere das im Zusammenhang mit dem erfindungsgemäßen Verfahren genannte System oder Teil dieses Systems sein. Das erfindungsgemäße Computerprogramm kodiert oder repräsentiert mit anderen Worten also die Verfahrensschritte zumindest einer Ausführungsform des erfindungsgemäßen Verfahrens. Das erfindungsgemäße Computerprogramm kann insbesondere zum Laden in einen elektronischen oder elektronisch lesbaren oder computerlesbaren Datenspeicher des Computers, insbesondere einer Datenverarbeitungseinrichtung einer Computertomographieanlage, ausgebildet und eingerichtet sein.

Ein weiterer Aspekt der vorliegenden Erfindung ist ein Datenträgersignal, welches zumindest eine Ausführungsform des erfindungsgemäßen Computerprogramms überträgt oder übertragen kann.

Ein weiterer Aspekt der vorliegenden Erfindung ist ein computerlesbares Speichermedium, also ein computerlesbarer Datenträger, auf dem zumindest eine Ausführungsform des erfindungsgemäßen Computerprogramms oder Computerprogrammprodukts gespeichert ist. Das erfindungsgemäße computerlesbare Speichermedium kann also insbesondere ein Datenträger für den genannten Computer, die genannte Datenverarbeitungseinrichtung, das genannte System oder die genannte Computertomographieanlage sein. Auf dem erfindungsgemäßen computerlesbaren Speichermedium oder Datenträger können weitere Befehle oder Steueranweisungen für den Computer, die Datenverarbeitungseinrichtung, das System oder die Computertomographieanlage gespeichert sein. Diese können beispielsweise dazu ausgebildet und eingerichtet sein, eine oder mehrere Abfragen an einen Benutzer oder Anwender zu erzeugen, entsprechende Benutzereingaben zu erfassen und/oder beispielsweise den genannten Hinweis beziehungsweise die genannte Warnung zu erzeugen.

Ein weiterer Aspekt der vorliegenden Erfindung ist ein System zum Bewerten einer Verlässlichkeit von CT-Volumenbildern eines Untersuchungsobjekts. Das erfindungsgemäße System umfasst eine Erfassungseinrichtung zum Erfassen eines aus gemessenen Projektionsbildern des Untersuchungsobjekts rechnerisch rekonstruierten ersten CT-Volumenbildes. Die Erfassungseinrichtung kann ebenso zum Erfassen eines zur Bildartefaktreduktion mittels eines Bildverbesserungsverfahrens erzeugten modifizierten CT-Volumenbildes desselben Untersuchungsobjekts eingerichtet sein. Die Erfassungseinrichtung kann dabei Teil einer Datenverarbeitungseinrichtung sein. Ebenso kann die Erfassungseinrichtung eine Aufnahmeeinrichtung einer Computertomographieanlage zum Aufnehmen, also zum Messen der gemessenen Projektionsbilder sein oder umfassen. Die Erfassungseinrichtung kann also beispielsweise den genannten Detektor und die genannte Strahlenquelle umfassen.

Weiter umfasst das erfindungsgemäße System eine mit der Erfassungseinrichtung verbundene Datenverarbeitungseinrichtung, welche zumindest eine Ausführungsform des erfindungsgemäßen computerlesbaren Speichermediums umfasst und zum Ausführen des darauf gespeicherten erfindungsgemäßen Computerprogramms eingerichtet ist. Das erfindungsgemäße System ist mit anderen Worten also zum Durchführen oder Ausführen zumindest einer Variante des erfindungsgemäßen Verfahrens eingerichtet. Dementsprechend kann das erfindungsgemäße System zumindest eine Auswahl der im Zusammenhang mit den übrigen Aspekten der vorliegenden Erfindung, also im Zusammenhang mit dem erfindungsgemäßen Verfahren, dem erfindungsgemäßen Computerprogramm und/oder dem erfindungsgemäßen computerlesbaren Speichermedium, genannten Eigenschaften und/oder Bauteile oder Komponenten aufweisen. Dies kann beispielsweise eine Ausgabeeinrichtung zum Ausgeben des genannten Hinweises oder der genannten Warnung oder eine Benutzerschnittstelle zum Empfangen oder Erfassen von Benutzereingaben oder dergleichen betreffen.

Zum Ausführen des erfindungsgemäßen Computerprogramms kann die Datenverarbeitungseinrichtung des erfindungsgemäßen Systems insbesondere einen Mikroprozessor, Mikrochip oder Mikrocontroller aufweisen, welche er mit dem computerlesbaren Speichermedium verbunden ist.

Das erfindungsgemäße System kann eine Computertomographieanlage sein. Ebenso kann das erfindungsgemäße System beispielsweise ein Computer oder eine Computereinrichtung sein, welche beispielsweise mit einer entfernt angeordneten Computertomographieanlage verbunden sein kann. Das erfindungsgemäße System, insbesondere dessen Datenverarbeitungseinrichtung, kann also Teil einer Computertomographieanlage sein aber ebenso beispielsweise in einem anderen Raum on-premise oder entfernt in einem Rechenzentrum angeordnet oder beispielsweise als Cloudserver realisiert sein.

Die bisher und im Folgenden angegebenen Eigenschaften und Weiterbildungen des erfindungsgemäßen Verfahrens, des erfindungsgemäßen Computerprogramms und des erfindungsgemäßen computerlesbaren Speichermediums sowie die entsprechenden Vorteile sind jeweils sinngemäß wechselseitig zwischen diesen Aspekten der Erfindung übertragbar. Es gehören also zu der Erfindung auch solche jeweils Weiterbildungen der Aspekte der vorliegenden Erfindung, welche Ausgestaltungen aufweisen, die hier zur Vermeidung unnötiger Redundanz nicht explizit in der jeweiligen Kombination oder nicht für jeden Aspekt der Erfindung separat beschrieben sind.

Weitere Merkmale, Einzelheiten und Vorteile der vorliegenden Erfindung ergeben sich aus der nachfolgenden Beschreibung bevorzugter Ausführungsbeispiele sowie anhand der Zeichnungen. Dabei zeigen:

FIG 1    schematisch ein computerlesbares Speichermedium mit einem schematisch angedeuteten Programmmodulen für ein Verfahrens zum Bewerten einer Verlässlichkeit eines CT-Volumenbildes;

FIG 2    diagrammatisch ein erstes Beispiel eines Verlaufs einer Likelihood-Funktion;

FIG 3    diagrammatisch ein zweites Beispiel eines Verlaufs einer Likelihood-Funktion; und

FIG 4    diagrammatisch ein drittes Beispiel eines Verlaufs einer Likelihood-Funktion;

Bei den im Folgenden erläuterten Ausführungsbeispielen handelt es sich um bevorzugte Ausführungsformen der Erfindung. Bei den Ausführungsbeispielen stellen die beschriebenen Komponenten der Ausführungsformen jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten Kombination als Bestandteil der Erfindung anzusehen sind. Des Weiteren sind die beschriebenen Ausführungsformen auch durch weitere der bereits beschriebenen Merkmale der Erfindung ergänzbar.

In den Figuren sind gleiche, funktionsgleiche oder einander entsprechende Details jeweils mit den gleichen Bezugszeichen gekennzeichnet.

In der Computertomographie können zur Artefaktreduktion und Bildverbesserung Methoden eingesetzt werden, welche Vorwissen beispielsweise in Form einer a-priori Verteilung oder trainierten neuronalen Netzen verwenden. Diese Methoden wurden in der Vergangenheit eher konservativ, also zurückhaltend eingesetzt. Ein Grund hierfür ist, dass sich das Vorwissen meist auf gesunde Menschen bezieht und nicht beispielsweise auf Pathologien oder Frakturen, da diese sehr vielfältig ausfallen können. Zusätzlich ist bei deep learning-basierten Verfahren schwer voraussagbar, ob diese tatsächlich in allen realen Situationen korrekt funktionieren werden, insbesondere dann, wenn die entsprechenden Situationen nicht während einer Trainingsphase trainiert wurden. Formal ausgedrückt kann es also problematisch sein, dass eine Bestimmung von Äquivalenzklassen bei einem Blackbox-Test nicht systematisch möglich ist.

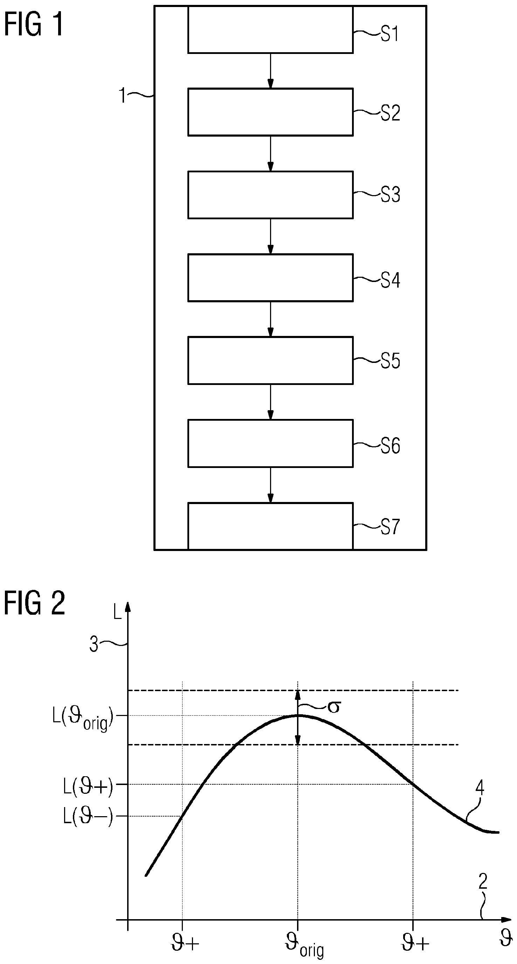

FIG 1 zeigt schematisch ein computerlesbares Speichermedium 1 mit einer Reihe von Blöcken S1 bis S7, welche Verfahrensschritte eines Verfahrens zum Bewerten einer Verlässlichkeit von CT-Volumenbildern eines Untersuchungsobjekts, vorliegend insbesondere eines Patienten, oder entsprechende Programmmodule eines dieses Verfahren implementierenden Computerprogramms oder Computerprogrammprodukts repräsentieren. Das hier vorgestellte Verfahren beziehungsweise das entsprechende Computerprogramm dient also zum Quantifizieren einer Verlässlichkeit oder Konfidenz von bestimmten Merkmalen eines CT-Volumenbildes, welches ein reales Volumen des Untersuchungsobjekts darstellt oder abbildet.

In dem Verfahrensschritt oder Block S1 werden dazu zweidimensionale Projektionsbilder des Untersuchungsobjekts mittels eines Computertomographen oder einer Computertomographieanlage erfasst.

In einem Verfahrensschritt oder Block S2 wird aus diesen Projektionsbildern ein erstes CT-Volumenbild rekonstruiert, also ein dreidimensionaler Datensatz, welcher das Untersuchungsobjekt abbildet, erzeugt. Dabei kann je nach Anwendungsfall bereits eine auf Vorwissen basierende Bildverbesserung eingesetzt werden.

In einem Verfahrensschritt oder Block S3 wird mittels eines automatischen Bildverbesserungsverfahrens eine Bildverbesserung oder Bildkorrektur des ersten CT-Volumenbildes durchgeführt und dadurch ein modifiziertes CT-Volumenbildern erzeugt. Dabei kann es sich insbesondere um lokale Modifikationen oder Realisierungsvarianten für bereits für Aspekte, also Details des ersten CT-Volumenbilds handeln.

In einem Verfahrensschritt oder Block S4 werden aus dem ersten CT-Volumenbild und aus dem modifizierten CT-Volumenbild digital rekonstruierte Röntgenbilder erzeugt, also simuliert oder berechnet.

Es ist dabei zu beachten, dass sowohl ein aus dem ersten CT-Volumenbild berechnetes digital rekonstruiertes Röntgenbild als auch ein aus dem modifizierten CT-Volumenbild berechnetes digital rekonstruiertes Röntgenbild sich von dem in der entsprechenden Projektionsrichtung aufgenommenen gemessenen Projektionsbild unterscheiden können. Die CT-Volumenbilder können also Artefakte oder Bildstörungen aufweisen. Diese können daraus resultieren, dass ein zum Rekonstruieren des jeweiligen CT-Volumenbildes verwendetes Modell beispielsweise in manchen Aspekten oder Annahmen nicht zu der Realität im Einzelfall, also zu der realen physischen Anatomie oder den realen physischen Eigenschaften des jeweiligen Untersuchungsobjekts und/oder nicht zu einer verwendeten Aufnahmemethode der gemessenen Projektionsbilder oder nicht zu einer Eigenschaft des verwendeten Computertomographen oder der verwendeten Röntgenstrahlung passt. Beispiele hierfür können etwa eine Strahlaufhärtung (englisch: Beam-Hardening) oder eine Bewegung des Untersuchungsobjekts während des Aufnehmens der gemessenen Projektionsbilder oder dergleichen sein.

In einem Verfahrensschritt oder Block S5 werden mittels einer Likelihood-Funktion eine Likelihood für das erste CT-Volumenbild und eine Likelihood für das modifizierte CT-Volumenbild unter Berücksichtigung der Ähnlichkeiten der digital rekonstruierten Röntgenbild zu dem oder den entsprechenden gemessenen Projektionsbildern berechnet. Dabei wird ein Likelihoodgradient, also ein Gradient der Likelihood-Funktion berechnet.

Weiter wird dann eine Differenz dieser berechneten Likelihoods mit einem Rauschen oder Rauschwert $\sigma$ des jeweiligen gemessenen Projektionsbildes verglichen oder in Beziehung gesetzt. Dabei sind verschiedene Konstellationen möglich, von denen einige beispielhaft in FIG 2, FIG 3 und FIG 4 illustriert sind. Darin ist jeweils diagrammatisch ein beispielhafter Verlauf der Likelihood-Funktion aufgetragen. Auf einer x-Achse oder Abszisse 2 ist dabei jeweils ein Parameter $\vartheta$ aufgetragen, welcher verschiedene Ausprägungen des rekonstruierten Volumens, also verschiedene Variationen oder Realisierungen eines durch das Bildverbesserungsverfahren modifizierten Merkmals angibt. $\vartheta_{orig}$ entspricht dabei der Realisierung in dem ersten CT-Volumenbild. $\vartheta+$ und $\vartheta-$ entsprechen verschiedenen möglichen Realisierungen des jeweils betrachteten Merkmals in dem modifizierten CT-Volumenbild beziehungsweise in verschiedenen modifizierten CT-Volumenbildern. Auf einer y-Achse oder Ordinate 3 ist jeweils die Likelihood L aufgetragen.

Bei dem in FIG 2 dargestellten ersten Verlauf 4 der Likelihood-Funktion steigt die Likelihood L von einem Wert L(d-) zu einem Wert L($\vartheta_{orig}$) an und fällt dann wieder ab hin zu einem Wert L($\vartheta+$). L($\vartheta-$) und L($\vartheta+$) entsprechen

dabei den Likelihoods verschiedener modifizierter CT-Volumenbilder und können auch als $L(\vartheta_{mod})$ bezeichnet werden. Da das erste CT-Volumenbild direkt aus den gemessenen Projektionsbildern ohne die für das modifizierte CT-Volumen verwendeten Bildverbesserungen erzeugt wurde, ist hier zusätzlich bei $\vartheta_{orig}$ eine Größe des Rauschwertes $\sigma$ der gemessenen Projektionsbilder beziehungsweise eine aufgrund von deren Rauschen zu erwartende Variation oder Unsicherheit der Likelihood L dargestellt. Bei dem in FIG 2 dargestellten ersten Verlauf 4 gilt sowohl für $\vartheta-$ als auch für $\vartheta+$, also zusammengefasst für $\vartheta_{mod}$, die Ungleichung

$$L(\vartheta_{orig}) - L(\vartheta_{mod}) > \sigma \quad .$$

Dies bedeutet oder wird so interpretiert, dass das jeweils betrachtete Merkmal in dem rekonstruierten ersten CT-Volumenbild durch die Messung, also die gemessenen Projektionsbilder bestätigt wird und die für die modifizierten CT-Volumenbilder vorgenommenen Modifizierungen oder Realisierungen $\vartheta-$, $\vartheta+$ dieses Merkmals eine geringere Likelihood L aufweisen, also mit geringerer Wahrscheinlichkeit der Realität entsprechen.

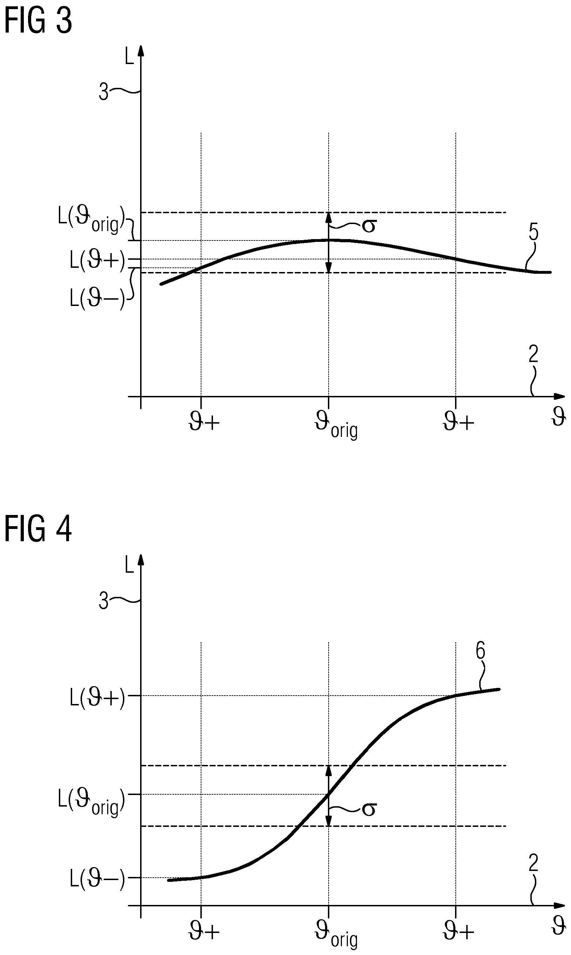

Ein in FIG 3 dargestellter zweiter Verlauf 5 der Likelihood-Funktion weist im Vergleich zu dem ersten Verlauf 4 eine geringere Krümmung oder Steigung auf. In diesem Fall sind die durch die Modifikationen für die modifizierten CT-Volumenbilder bewirkten Veränderungen der Likelihood gegenüber der Likelihood des ersten CT-Bildes beziehungsweise der darin enthaltenen Realisierung des jeweiligen betrachteten Merkmal geringer als der Rauschwert o. Dementsprechend gilt hier die Ungleichung

$$| L(\vartheta_{orig}) - L(\vartheta_{mod}) | \leq \sigma \quad .$$

Die Modifikationen des Merkmals beziehungsweise der Realisierung $\vartheta$ des betrachteten Merkmals führt also zu keiner signifikanten Veränderung der Likelihood. Das bedeutet oder wird so interpretiert, dass keine Signifikanz für das betrachtete Merkmal in der Messung, also in den gemessenen Projektionsbildern, gefunden werden kann.

In FIG 4 ist ein dritter Verlauf 6 der Likelihood-Funktion dargestellt. Hier steigt die Likelihood von dem Wert L(d-) für eine erste modifizierte Realisierung $\vartheta$ des betrachteten Merkmals hin zu einem Wert $L\vartheta_{orig})$ für die Realisierung $\vartheta_{orig}$ in dem ersten CT-Volumenbild und weiter bis zu einem Wert $L(\vartheta+)$ für eine zweite Realisierung $\vartheta+$ in einem zweiten modifizierten CT-Volumenbild an. Dabei ist also die Likelihood $L(\vartheta+)$ für die zweite Realisierung $\vartheta+$ größer als die Likelihood $L(\vartheta_{orig})$ die Realisierung $\vartheta$ des ersten CT-Volumenbildes, sodass für die zweite modifizierte Realisierung $\vartheta+$ die Ungleichung

$$L(\vartheta+) - L(\vartheta_{orig}) > \sigma$$

gilt. Die zweite Veränderung oder Modifikation, also die Realisierung $\vartheta+$, führt also zu einer Erhöhung oder einem Anstieg der Likelihood L für das entsprechend modifizierte CT-Volumenbild gegenüber dem ersten CT-Volumenbild. Dies bedeutet oder wird so interpretiert, dass die Messung, also die gemessenen Projektionsbilder, dem rekonstruierten ersten CT-Volumenbild beziehungsweise der darin vorgesehenen Realisierung $\vartheta_{orig}$ des betrachteten Merkmals widerspricht. Dementsprechend kann dann bevorzugt das zweite modifizierte CT-Volumenbild mit der Realisierung $\vartheta+$ als Grundlage beispielsweise für eine Bewertung oder Diagnose verwendet werden.

Auf entsprechende Weise können die Likelihoods L für jedes modifizierte Merkmal, also für eine Vielzahl von modifizierten Voxeln der CT-Volumenbilder berechnet und miteinander verglichen werden.

In einem Verfahrensschritt oder Block S6 wird anhand der bestimmten Likelihood eine Konfidenzkarte und/oder ein globaler Konfidenz- oder Verlässlichkeitswert erzeugt beziehungsweise bestimmt. Die Konfidenzkarte beziehungsweise der Konfidenz- oder Verlässlichkeitswert kann dann einem Anwender als Hinweis angezeigt und/oder als Entscheidungskriterium genutzt werden, um beispielsweise automatisch oder halbautomatisch zu entscheiden oder zu bestimmen, ob ein für das Erzeugen des oder der modifizierten CT-Volumenbilder verwendetes oder verwendbares Bildverbesserungsverfahren angeboten oder verwendet werden soll oder nicht.

In einem Verfahrensschritt oder Block S7 kann ein Vergleich des oder der bestimmten Likelihoods, Konfidenz- oder Verlässlichkeitswerte mit einem entsprechenden vorgegebenen Schwellenwert automatisch durchgeführt werden. Wird dabei festgestellt, dass der vorgegebene Schwellenwert unterschritten wird, so kann automatisch ein entsprechender Hinweis oder eine entsprechende Warnung erzeugt und an einen jeweiligen Benutzer ausgegeben werden.

Insgesamt zeigen die beschriebenen Beispiele, wie vorteilhaft eine Praxistauglichkeit eines Verfahrens zur automatischen Verbesserung einer Bildqualität verbessert werden kann.

**Patentansprüche**

1.  Verfahren (S1-S7) zum Bewerten einer Verlässlichkeit von CT-Volumenbildern eines Untersuchungsobjekts, wobei

    - ein aus gemessenen Projektionsbildern des Untersuchungsobjekts rechnerisch rekonstruiertes erstes CT-Volumenbild erfasst wird,
    - ein zur Bildartefaktreduktion mittels eines Bildverbesserungsverfahrens erzeugtes modifiziertes CT-Volumenbild desselben Untersuchungsobjekts erfasst wird,

- wenigstens eine Projektionsrichtung bestimmt wird, für die ein simuliertes Strahlenbündel wenigstens ein im Vergleich zu dem ersten CT-Volumenbild modifiziertes Voxel des modifizierten CT-Volumenbildes durchdringt,

- für die wenigstens eine bestimmte Projektionsrichtung aus dem ersten CT-Volumenbild und aus dem modifizierten CT-Volumenbild jeweils wenigstens ein digital rekonstruiertes Röntgenbild berechnet wird,

- jeweils eine Ähnlichkeit bestimmt wird zwischen dem wenigstens einen aus dem ersten CT-Volumenbild berechneten digital rekonstruierten Röntgenbild und dem jeweils entsprechenden gemessenen Projektionsbild einerseits und zwischen dem wenigstens einen aus dem modifizierten CT-Volumenbild berechneten digital rekonstruierten Röntgenbild und dem jeweils entsprechenden gemessenen Projektionsbild andererseits, und

- zum Bewerten der Verlässlichkeit der CT-Volumenbilder die bestimmten Ähnlichkeiten miteinander verglichen werden.

2. Verfahren nach Anspruch 1, **dadurch gekennzeichnet, dass** bereits das erste CT-Volumenbild als durch auf Vorwissen basierende Abschätzungen für eine verbesserte Bildqualität angepasstes CT-Volumenbild rekonstruiert wird und das modifizierte CT-Volumenbild durch lokale Modifikationen aus dem ersten CT-Volumenbild erzeugt wird.

3. Verfahren nach Anspruch 1, **dadurch gekennzeichnet, dass** zum Rekonstruieren des ersten CT-Volumenbildes nur direkt in den gemessenen Projektionsdaten enthaltene Informationen und keine darüber hinausgehenden Abschätzungen verwendet werden, derartige Abschätzungen aber zum Erzeugen des modifizierten CT-Volumenbildes durch das Bildverbesserungsverfahren angewendet werden.

4. Verfahren (S1-S7) nach einem der vorhergehenden Ansprüche, **dadurch gekennzeichnet, dass** die Ähnlichkeiten mittels einer Likelihood-Funktion (L, 4, 5, 6) charakterisiert werden und zum Bewerten der Verlässlichkeit mittels der Likelihood-Funktion (L, 4, 5, 6) jeweils eine Likelihood ($L(\vartheta_{orig})$) für das erste CT-Volumen und eine Likelihood ($L(\vartheta_+)$, $L(\vartheta_-)$) für das modifizierte CT-Volumen berechnet wird, wobei die Likelihood ($L(\vartheta_{orig})$, $L(\vartheta_+)$, $L(\vartheta_-)$) für ein CT-Volumenbild maximal wird wenn ein daraus berechnetes digital rekonstruiertes Röntgenbild und das entsprechende gemessene Projektionsbild identisch sind.

5. Verfahren (S1-S7) nach Anspruch 4, **dadurch gekennzeichnet, dass** ein Rauschen $\sigma$ des wenigstens einen gemessenen Projektionsbildes bestimmt wird und die Verlässlichkeit in Abhängigkeit davon bewertet wird, ob eine Differenz zwischen der für das erste CT-Volumenbild berechneten Likelihood $L(\vartheta_{orig})$ und der für das modifizierte CT-Volumenbild berechneten Likelihood $L(\vartheta_{mod})$ größer oder kleiner als das Rauschen $\sigma$ ist.

6. Verfahren (S1-S7) nach einem der vorhergehenden Ansprüche, **dadurch gekennzeichnet, dass** für das modifizierte CT-Volumenbild und/oder für das erste CT-Volumenbild eine Konfidenzkarte erzeugt wird, welche zumindest für jedes durch das Bildverbesserungsverfahren modifizierte Voxel dessen Verlässlichkeit angibt.

7. Verfahren (S1-S7) nach Anspruch 6, **dadurch gekennzeichnet, dass** die in der Konfidenzkarte eingetragenen Verlässlichkeiten nach einem vorgegebenen Schema gemäß ihren Größen farbcodiert werden.

8. Verfahren (S1-S7) nach einem der vorhergehenden Ansprüche, **dadurch gekennzeichnet, dass** automatisch ein Hinweis an einen Benutzer ausgegeben wird, falls die Verlässlichkeit kleiner als ein vorgegebene Schwellenwert ist.

9. Verfahren (S1-S7) nach einem der vorhergehenden Ansprüche, **dadurch gekennzeichnet, dass** durch ein zum Durchführen eines Verfahrens (S1-S7) nach einem der vorhergehenden Ansprüche eingerichtetes System automatisch eine von dem verwendeten Bildverbesserungsverfahren verschiedene Methode zur Bildoptimierung verwendet oder vorgeschlagen wird, falls die Verlässlichkeit des modifizierten CT-Volumenbildes kleiner als ein vorgegebene Schwellenwert ist.

10. Computerprogramm (S1-S7), umfassend Befehle, die bei einer Ausführung des Computerprogramms (S1-S7) durch einen Computer diesen dazu veranlassen, ein Verfahren (S1-S7) nach einem der vorhergehenden Ansprüche auszuführen.

11. Computerlesbares Speichermedium (1), auf dem ein Computerprogramm (S1-S7) nach Anspruch 10 gespeichert ist.

12. System zum Bewerten einer Verlässlichkeit von CT-Volumenbildern eines Untersuchungsobjekts, umfassend

- eine Erfassungseinrichtung zum Erfassen eines aus gemessenen Projektionsbildern des Untersuchungsobjekts rechnerisch rekonstruierten ersten CT-Volumenbildes, und
- eine mit der Erfassungseinrichtung verbundene Datenverarbeitungseinrichtung, welche ein computerlesbares Speichermedium (1) nach Anspruch 11 umfasst und zum Ausführen des darauf gespeicherten Computerprogramms (S1-S7) eingerichtet ist.

**Claims**

1. Method (S1-S7) for evaluating a reliability of CT volume images of an examination object, wherein

   - a computationally reconstructed first CT volume image is acquired from scanned projection images of the examination object,
   - a modified CT volume image of the same examination object created for image artefact reduction by means of an image enhancement method is acquired,
   - at least one projection direction is determined for which a simulated radiation beam penetrates at least one voxel of the modified CT volume image, said voxel being modified in comparison with the first CT volume image,
   - for the at least one determined projection direction, respectively at least one digitally reconstructed X-ray image is calculated from the first CT volume image and from the modified CT volume image,
   - a similarity is determined, in each case, between the at least one digitally reconstructed X-ray image calculated from the first CT volume image and the respective corresponding scanned projection image, on the one hand, and between the at least one digitally reconstructed X-ray image calculated from the modified CT volume image and the respective corresponding scanned projection image, on the other hand, and
   - for evaluating the reliability of the CT volume images, the determined similarities are compared with one another.

2. Method according to claim 1, **characterised in that** the first CT volume image is already reconstructed as a CT volume image adapted through estimates on the basis of prior knowledge for an improved image quality and the modified CT volume image is generated by local modifications from the first CT volume image.

3. Method according to claim 1, **characterised in that** for the reconstruction of the first CT volume image, only information contained directly in the scanned projection data and no estimates extending therebeyond are used, but such estimates are used for generating the modified CT volume image by means of the image enhancement method.

4. Method (S1-S7) according to one of the preceding claims, **characterised in that** the similarities are **characterised by** means of a likelihood function (L, 4, 5, 6) and for the evaluation of the reliability by means of the likelihood function (L, 4, 5, 6), in each case, a likelihood ($L(\vartheta_{orig})$) is calculated for the first CT volume and a likelihood ($L(\vartheta_{+})$, $L(\vartheta-)$) is calculated for the modified CT volume, wherein the likelihood ($L(\vartheta_{orig})$, $L(\vartheta_{+})$, $L(\vartheta-)$) for a CT volume image is a maximum if a digitally reconstructed X-ray image calculated therefrom and the corresponding scanned projection image are identical.

5. Method (S1-S7) according to claim 4, **characterised in that** a noise $\sigma$ of the at least one scanned projection image is determined and the reliability is then evaluated dependent upon whether a difference between the likelihood $L(\vartheta_{orig})$ calculated for the first CT volume image and the likelihood ($\vartheta_{mod}$) calculated for the modified CT volume image is larger or smaller than the noise $\sigma$.

6. Method (S1-S7) according to one of the preceding claims, **characterised in that** for the modified CT volume image and/or for the first CT volume image, a confidence map is generated which shows at least for each voxel modified by the image enhancement method, its reliability.

7. Method (S1-S7) according to claim 6, **characterised in that** the reliabilities entered in the confidence map are color-coded according to a pre-defined scheme depending upon their sizes.

8. Method (S1-S7) according to one of the preceding claims, **characterised in that** an indication is issued automatically to a user if the reliability is smaller than a pre-determined threshold value.

9. Method (S1-S7) according to one of the preceding claims, **characterised in that** by means of a system configured to carry out a method (S1-S7) according to one of the preceding claims, a method for image optimisation different from the image enhancement method used is automatically used or proposed if the reliability of the modified CT volume image is smaller than a pre-determined threshold value.

10. Computer program (S1-S7) comprising commands which, on an execution of the computer program (S1-S7) by a computer, cause it to carry out a method (S1-S7) according to one of the preceding claims.

11. Computer-readable storage medium (1) on which a computer program (S1-S7) according to claim 10 is stored.

12. System for evaluating a reliability of CT volume images of an examination object, comprising

- an acquisition device for acquiring a computationally reconstructed first CT volume image from scanned projection images of the examination object, and
- a data processing device connected to the acquisition device, which comprises a computer-readable storage medium (1) according to claim 11 and is configured for carrying out the computer program (S1-S7) stored thereon.

## Revendications

1. Procédé (S1 à S7) d'évaluation de la fiabilité d'images de volume CT d'un objet à examiner, dans lequel

- on prend une première image de volume CT reconstruite par le calcul à partir d'images de projection mesurées,
- on prend une image de volume CT modifiée et produite pour la réduction d'artefact d'image au moyen d'un procédé d'amélioration d'image, du même objet à examiner,
- on détermine au moins une direction de projection, pour laquelle un faisceau de rayonnement simulé traverse au moins un voxel, modifié par rapport à la première image de volume CT, de l'image de volume CT modifiée,
- pour la au moins une direction de projection déterminée, on calcule, à partir de la première image de volume CT et à partir de l'image de volume CT modifiée, respectivement au moins une image de rayon X reconstruite numériquement,
- on détermine respectivement une similitude entre la au moins une image de rayon X calculée à partir de la première image de volume CT et reconstruite numériquement et l'image de projection mesurée correspondante, d'une part, et entre la au moins une image de rayon X calculée à partir de l'image de volume CT modifiée et reconstruite numériquement et l'image de projection mesurée correspondante, d'autre part, et
- pour l'évaluation de la fiabilité des images de volume CT, on compare entre elles les similitudes déterminées.

2. Procédé suivant la revendication 1, **caractérisé en ce que** l'on reconstruit déjà la première image de volume CT sous la forme d'une image de volume CT adaptée par des estimations reposant sur des savoirs antérieurs d'une amélioration de la qualité de l'image et **en ce que** l'on produit l'image de volume CT modifiée par des modifications locales à partir de la première image de volume CT.

3. Procédé suivant la revendication 1, **caractérisé en ce que** l'on n'utilise, pour la reconstruction de la première image de volume CT, que seulement des informations contenues directement dans les données de projection mesurées et pas d'estimations allant au-delà, mais on applique des estimations de ce genre pour la projection de l'image de volume CT modifiée par le procédé d'amélioration d'image.

4. Procédé (S1 à S7) suivant l'une des revendications précédentes, **caractérisé en ce que** l'on caractérise les similitudes au moyen d'une fonction (L, 4, 5, 6) de probabilité et on calcule, pour l'évaluation de la fiabilité, au moyen de la fonction (L, 4, 5, 6) de probabilité, respectivement une probabilité ($L(\vartheta_{orig})$) du premier volume CT et une probabilité ($L(\vartheta_+)$), ($L(\vartheta_-)$) du volume CT modifié, la probabilité ($L(\vartheta_{orig})$), ($L(\vartheta_+)$), ($L(\vartheta_-)$) de l'image de volume CT étant maximum, lorsqu'une image de rayon X reconstruite numériquement, qui en est calculée, et l'image de projection mesurée correspondante sont identiques.

5. Procédé (S1 à S7) suivant la revendication 4, **caractérisé en ce que** l'on détermine un bruit $\sigma$ d'au moins une image de projection mesurée et on évalue la fiabilité en fonction du point de savoir si une différence entre la probabilité ($L(\vartheta_{orig})$) calculée pour la première image de volume CT et la probabilité $L(\vartheta_{mod})$ calculée pour l'image de volume CT modifiée est plus grande ou plus petite que le bruit $\sigma$.

6. Procédé (S1 à S7) suivant l'une des revendications précédentes, **caractérisé en ce que** l'on produit, pour l'image de volume CT modifiée et/ou pour la première image de volume CT, une carte de confiance, qui indique, au moins pour chaque voxel modifié par le procédé d'amélioration de l'image, sa fiabilité.

7. Procédé (S1 à S7) suivant la revendication 6, **caractérisé en ce que** l'on code en couleur, suivant un schéma donné à l'avance selon leur dimension, les fiabilités portées dans la carte de confiance.

8. Procédé (S1 à S7) suivant l'une des revendications précédentes, **caractérisé en ce que** l'on émet automatiquement une indication à un utilisateur si la fiabilité est plus petite qu'une valeur de seuil donnée à l'avance.

9. Procédé (S1 à S7) suivant l'une des revendications précédentes, **caractérisé en ce que** l'on utilise ou l'on propose pour l'optimisation d'image, par un système conçu pour effectuer un procédé (S1 à S7) suivant l'une des revendications précédentes, automatiquement une méthode différente du procédé d'amélioration image utilisé, si la fiabilité de l'image de volume CT modifiée est plus petite qu'une valeur de seuil donnée à l'avance.

10. Programme (S1 à S7) d'ordinateur, comprenant des instructions, qui, lors d'une réalisation du programme (S1 à S7) d'ordinateur par un ordinateur, font que celles-ci effectuent un procédé (S1 à S7) suivant l'une des revendications précédentes.

11. Support (1) de mémoire déchiffrable par ordinateur, sur lequel est mis en mémoire un programme (S1 à S7) d'ordinateur suivant la revendication 10.

12. Système d'évaluation d'une fiabilité d'image de volume CT d'un objet à examiner, comprenant

    - un dispositif de prise d'une première image de volume CT reconstruite informatiquement à partir d'images de projection mesurées de l'objet à examiner, et
    - un dispositif de traitement de données, qui est relié au dispositif de prise, qui comprend un support (1) de mémoire déchiffrable par ordinateur suivant la revendication 11 et qui est conçu pour effectuer le programme (S1 à S7) d'ordinateur qui y est mis en mémoire.

FIG 1

FIG 2

FIG 3

FIG 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- Globally Convergent Algorithms for Maximum a Posteriori Transmission Tomography. **LANGE ; FESSLER.** IEEE TRANSACTIONS ON IMAGE PROCESSING. IEEE SERVICE CENTER, Oktober 1995, vol. 4, 1430-1438